# DEMANDE DE BREVET EUROPEEN

(11) **EP 1 190 700 A1**
(43) Date de publication de la demande: **27.03.2002**
(21) Numéro de dépôt: 01402396.4
(22) Date de dépôt: 19.09.2001
(51) Int. Cl.: A61K 7/11

(54) **Composition comprenant des nanoparticules d'alumine et au moins un polymère fixant**

(30) Priorité: 20.09.2000 FR 0011992
(71) Demandeur: L'OREAL, 75008 Paris (FR)
(72) Inventeur: Nocerino, Cécile, 75006 Paris (FR); Giroud, Franck, 92110 Clichy (FR); Sturlag, Jean-Michel, 92100 Boulogne (FR)
(74) Mandataire: Casalonga, Axel

(57) **Abrégé**

La présente invention concerne une composition qui comprend au moins un polymère fixant et des nanoparticules contenant au moins 10 % en poids d'alumine et présentant une taille primaire moyenne en nombre inférieure à 25 nm. L'invention concerne aussi un procédé de traitement cosmétique des cheveux mettant en oeuvre la composition selon l'invention, ainsi qu'une utilisation de la composition comme produit de coiffage.

## Description

La présente invention est relative à une composition comprenant, dans un milieu cosmétiquement acceptable, au moins un polymère fixant et des nanoparticules d'alumine, à un procédé de traitement cosmétique des cheveux, et à une utilisation comme produit de coiffage.

Des produits tels que, par exemple, des laques, des mousses et des gels, sont bien connus dans la technique pour coiffer les cheveux en les fixant entre eux.

Cependant, un simple passage des doigts, d'un peigne ou d'une brosse entraîne une cassure des soudures créées lors de l'application de ces produits classiques. Ils ne permettent donc pas un recoiffage des cheveux.

Le brevet US 3 819 827 de WELLA décrit en particulier des produits pour la mise en plis des cheveux comprenant de 0,2 à 6 % en poids de particules d'oxyde d'aluminium présentant une taille de particule d'environ 30 mµ, et de 1 à 4 % en poids de polymères tels que, par exemple, la gomme adragante, l'agar-agar, la pectine, des polymères vinyliques et des polymères basiques.

La Demanderesse a trouvé de manière surprenante et inattendue que l'utilisation de nanoparticules contenant au moins 10 % en poids d'alumine et présentant une taille primaire moyenne en nombre inférieure à 25 nm, en association avec un polymère fixant, dans un milieu cosmétiquement acceptable, permettait de donner du volume à la chevelure et d'obtenir un coiffage sans fixer et surcharger les cheveux. Les cheveux peuvent alors être coiffés et recoiffés à volonté tout en conservant un aspect naturel. En outre, cette association permet d'obtenir plus de douceur et un démêlage nettement meilleur.

Par milieu cosmétiquement acceptable, on entend un milieu compatible avec les cheveux, mais aussi d'odeur, d'aspect et de toucher agréables.

L'invention a donc pour objet une composition qui comprend, dans un milieu cosmétiquement acceptable, au moins un polymère fixant et des nanoparticules contenant au moins 10 % en poids d'alumine et présentant une taille primaire moyenne en nombre inférieure à 25 nm.

Un autre objet de la présente invention consiste en un procédé de traitement cosmétique des cheveux mettant en oeuvre la composition selon l'invention.

L'invention a encore pour objet une utilisation de la composition selon l'invention, comme produit de coiffage.

D'autres objets, caractéristiques, aspects et avantages de l'invention apparaîtront encore plus clairement à la lecture de la description et des divers exemples qui suivent.

Selon l'invention, la composition cosmétique comprend, dans un milieu cosmétiquement acceptable, au moins un polymère fixant et des nanoparticules contenant au moins 10 % en poids d'alumine et présentant une taille primaire moyenne en nombre inférieure à 25 nm, de préférence comprise entre 5 et 25 nm.

Les particules selon l'invention peuvent, par exemple, avoir une forme quelconque, par exemple la forme de sphères, de paillettes, d'aiguilles, de plaquettes ou des formes totalement aléatoires. Les particules convenant particulièrement dans l'invention ont une forme essentiellement sphérique.

Au sens de la présente invention, on entend par "taille primaire de particule", la dimension maximale qu'il est possible de mesurer entre deux points diamétralement opposés d'une particule individuelle. Cette taille peut être déterminée par microscopie électronique ou à partir de la mesure de la surface spécifique par la méthode BET.

Conformément à la présente invention, les particules solides contenant de l'alumine forment une masse dans laquelle l'alumine ne sert pas d'agent d'enrobage à une (ou d'autres) charge(s).

Dans le cas où les nanoparticules sont formées par de l'alumine et d'autres charges, l'alumine se trouve à l'état libre et ne forme pas de liaisons chimiques avec les autres charges. Il s'agit alors d'un alliage entre l'alumine et d'autres charges, notamment avec des oxydes de métaux ou de métalloïdes, en particulier obtenu par fusion thermique de ces différents constituants.

Lorsque les nanoparticules contenant au moins 10 % en poids d'alumine comprennent en outre un oxyde de métal ou de métalloïde autre que l'alumine, celui-ci est notamment choisi parmi l'oxyde de silicium ou de bore.

De préférence, les nanoparticules contiennent au moins 50 % en poids d'alumine, de préférence au moins 70 % en poids, et les nanoparticules constituées à plus de 90 % en poids d'alumine sont particulièrement préférées selon la présente invention.

L'alumine convenant dans les compositions de la présente invention est de préférence une alumine éventuellement hydratée, telle que, par exemple, la boehmite.

Les nanoparticules contenant au moins 10 % en poids d'alumine selon l'invention peuvent être utilisées en une quantité comprise entre 0,01 % et 30 %, et de préférence entre 0,05 % et 5 % en poids par rapport au poids total de la composition.

La composition peut également contenir d'autres types de nanoparticules, par exemple d'oxyde de titane ou de zinc.

Les polymères fixants convenant dans l'invention sont ceux généralement utilisés dans la technique. Ils sont notamment choisis parmi les polymères cationiques, anioniques, amphotères, non ioniques et leurs mélanges.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants
(1) les homopolymères ou copolymères dérivés d'esters ou d'amides acryliques ou méthacryliques et comportant au moins un des motifs de formules suivantes : 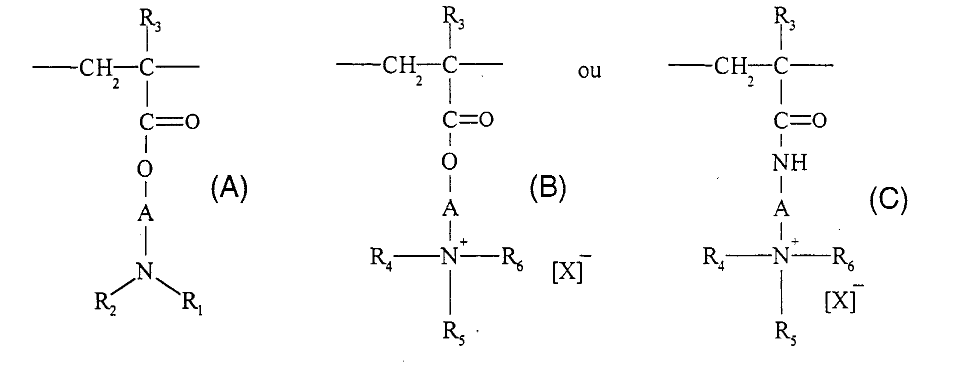 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non, tels que les produits vendus sous la dénomination "GAFQUAT® " par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français 2 077 143 et 2 393 573,
   - les terpolymères méthacrylate de diméthylaminoéthyle/ vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP.
(2) les polysaccharides quaternisés tels que les gommes de guar ;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL .

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 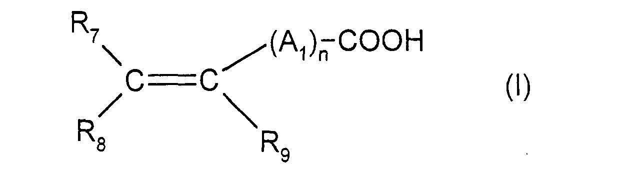 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique
   ou leurs sels et en particulier les produits vendus sous les dénominations VERSICOL® E ou K par la société ALLIED COLLOID et ULTRAHOLD® par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée. comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique et les copolymères acétate de vinyle/acide crotonique greffés par du polyéthylèneglycol vendus sous la dénomination ARISTOFLEX® A par la société BASF.

Les polymères fixants anioniques les plus particulièrement préférés sont choisis parmi les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques,
ou bien B et C peuvent désigner des groupements dérivant de monomères zwittérioniques de carboxybétaïnes ou de sulfobétaïnes ;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides
      ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale :

   ⁅CO-R₁₀-CO-Z⁆ (II)

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylènepolyamine bis-primaire, mono- ou bis-secondaire et de préférence représente :
   a) dans les proportions de 60 à 100 % en moles, le groupe 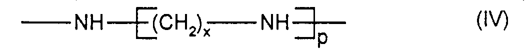 où x=2 et p=2 ou 3, ou bien x=3 et p=2
      ce groupe dérivant de la diéthylène-triamine, de la triéthylènetétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 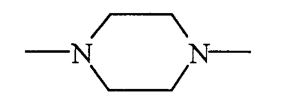
   c) dans les proportions de 0 à 20 % en moles, le groupe -NH-(CH₂)₆-NH- dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 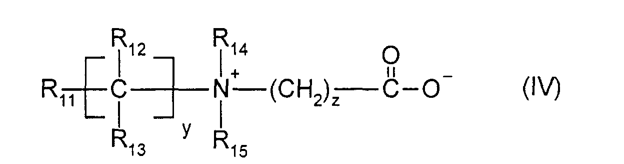 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle.
(5) les polymères dérivés du chitosane comportant des motifs monomères répondant aux formules suivantes : 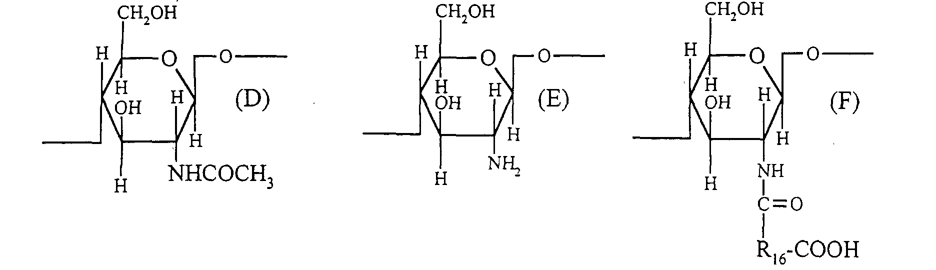 le motif (D) étant présent dans des proportions comprises entre 0 et 30%; le motif (E) dans des proportions comprises entre 5 et 50% et le motif (F) dans des proportions comprises entre 30 et 90%, étant entendu que dans ce motif (F), R₁₆ représente un groupe de formule : 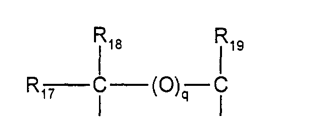 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases
   ou des acides.
(6) Les polymères répondant à la formule générale (V) sont, par exemple, décrits dans le brevet français 1 400 366 : 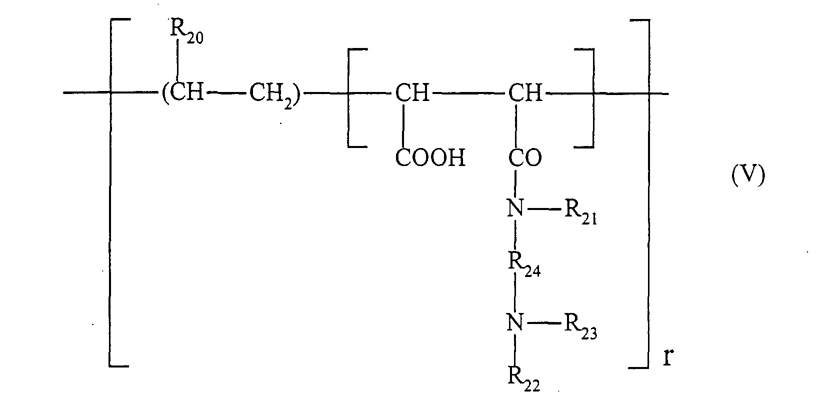 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 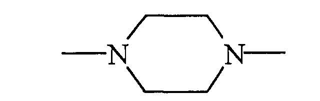 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 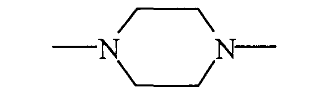 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer, tels que les produits vendus sous les dénominations AMPHOMER® , AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ;
- les copolymères de styrène, de méthacrylate d'alkyle et d'acrylate d'alkyle ;
- les polyuréthannes
- les copolymères de styrène et de butadiène ;
- les copolymères de styrène, de butadiène et de vinylpyridine ;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères fixants sont de préférence des polymères non-ioniques, et mieux encore des polymères non ioniques à motifs vinyllactames. Ils sont notamment décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.

Les homopolymères ou copolymères à motifs vinyllactame comprennent des motifs de formule : 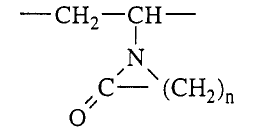 dans laquelle n est indépendamment 3, 4 ou 5.

La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

Les polymères fixants, particulièrement préférés dans la présente invention, sont les polyvinylpyrrolidones telles que celles commercialisées sous la dénomination Luviskol® K30 par la société BASF ; les polyvinylcaprolactames tels que ceux commercialisés sous la dénomination Luviskol® PLUS par la société BASF ; les copolymères poly(vinylpyrrolidone/acétate de vinyle) tels que ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol® VAP 343 par la société BASF.

Les polymères fixants peuvent être utilisés en une quantité comprise entre 0,01 % et 20 % en poids, de préférence entre 0,1% et 10 % en poids par rapport au poids total de la composition selon l'invention.

Le milieu cosmétiquement acceptable comprend de l'eau et/ou un solvant cosmétiquement acceptable. Ce solvant cosmétiquement acceptable est notamment choisi parmi les alcools inférieurs en C₁-C₄ comme l'éthanol, l'isopropanol, le tertio-butanol, le n-butanol ; les polyols comme le propylèneglycol, et les éthers de polyols ; l'acétone ; et leurs mélanges, le solvant particulièrement préféré étant l'éthanol.

La composition selon l'invention peut comprendre en outre des additifs cosmétiques usuels choisis parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les tensioactifs, les silicones volatiles ou non, les huiles végétales, animales, minérales,
ou de synthèse, les protéines et les vitamines, et leurs mélanges.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions conformes à l'invention peuvent se présenter sous forme d'un gel, d'un liquide ou d'une mousse et être utilisées en application rincée ou non. Elle peuvent être conditionnées dans un dispositif aérosol usuel en cosmétique.

Les compositions conformes à l'invention peuvent être utilisées en tant que compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

La présente invention concerne également un procédé de traitement cosmétique des cheveux qui consiste à appliquer une quantité efficace d'une composition telle que décrite ci-dessus, sur les cheveux, à rincer ou non après un éventuel temps de pose.

Selon un mode de réalisation préféré de l'invention, la composition peut être utilisée comme produit de coiffage non rincé.

L'exemple suivant illustre la présente invention et ne doit être considéré en aucune manière comme limitant l'invention.

### Exemple

On a préparé un produit de coiffage à partir des ingrédients suivants. Les pourcentages indiqués sont des pourcentages en poids.

| | |
|---|---|
| Oxyde d'aluminium⁽¹⁾ | 0,3 % |
| Polyvinylpyrrolidone | 2 % |
| Alcool éthylique | 32,7 % |
| Diméthyléther | 65 % |

| | |
|---|---|
| ⁽¹⁾ Vendu sous la dénomination ALUMINIUMOXID C par la société DEGUSSA-HULS, et présentant une taille primaire moyenne en nombre de 13 nm. | |

On mélange les ingrédients dans un bidon muni d'une valve et d'un diffuseur. On pulvérise cette composition sur les cheveux et on laisse sécher quelques secondes.

Les cheveux présentent beaucoup de volume, sont lisses et faciles à coiffer et à recoiffer.

## Revendications

1. Composition **caractérisée en ce qu'**elle comprend, dans un milieu cosmétiquement acceptable, au moins un polymère fixant et des nanoparticules contenant au moins 10 % en poids d'alumine et présentant une taille primaire moyenne en nombre inférieure à 25 nm.

2. Composition selon la revendication 1, **caractérisée en ce que** les nanoparticules contenant au moins 10 % en poids d'alumine comprennent en outre un oxyde de métal ou de métalloïde autre que l'alumine.

3. Composition selon la revendication 2, **caractérisée en ce que** l'oxyde de métal ou de métalloïde est choisi parmi l'oxyde de silicium, de bore.

4. Composition selon l'une quelconque des revendications précédentes, **caractérisée en ce que** les nanoparticules contiennent au moins 50 % en poids d'alumine.

5. Composition selon l'une quelconque des revendications précédentes, **caractérisée en ce que** les nanoparticules sont constituée à plus de 90 % en poids d'alumine.

6. Composition selon l'une quelconque des revendications précédentes, **caractérisée en ce que** l'alumine est une alumine éventuellement hydratée.

7. Composition selon la revendication 6, **caractérisée en ce que** l'alumine est la boehmite.

8. Composition selon l'une quelconque des revendications précédentes, **caractérisée en ce que** la quantité des nanoparticules est comprise entre 0,01 et 30 % en poids, de préférence entre 0,05 et 5 % en poids par rapport au poids de la composition.

9. Composition selon l'une quelconque des revendications précédentes, **caractérisée en ce que** le polymère fixant est un polymère non-ionique.

10. Composition selon la revendication 9, **caractérisée en ce que** le polymère fixant non-ionique est un homopolymère ou copolymère à motifs vinyllactame, qui comprend des motifs de formule : 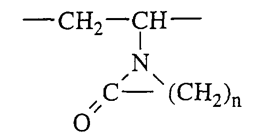 dans laquelle n est indépendamment 3, 4 ou 5.

11. Composition selon la revendication 9 ou 10, caractérisée en ce le polymère fixant est choisi parmi les polyvinylpyrrolidones, les polyvinylcaprolactames, les poly(vinylpyrrolidone/acétate de vinyle) et les poly(vinylpyrrolidone/acétate de vinyle/propionate de vinyle).

12. Composition selon l'une quelconque des revendications précédentes, **caractérisée en ce que** le polymère fixant est contenu en une quantité comprise entre 0,01 % et 20 % en poids, de préférence entre 0,1 % et 10 % en poids par rapport au poids total de la composition.

13. Composition selon l'une quelconque des revendications précédentes, **caractérisée en ce que** le milieu cosmétiquement acceptable comprend de l'eau et/ou un solvant cosmétiquement acceptable.

14. Composition selon la revendication 13, **caractérisée en ce que** le solvant cosmétiquement acceptable est choisi parmi les alcools inférieurs en C₁-C₄, les polyols, les éthers de polyols, l'acétone et leurs mélanges.

15. Composition selon la revendication 14, **caractérisée en ce que** le solvant cosmétiquement acceptable est l'éthanol.

16. Composition selon l'une quelconque des revendications précédentes, **caractérisée en ce qu'**elle comprend en outre des additifs cosmétiques usuels choisis parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les tensioactifs, les silicones volatiles ou non, les huiles végétales, animales ou minérales, les protéines et les vitamines, et leurs mélanges.

17. Composition selon l'une quelconque des revendications précédentes, **caractérisée en ce qu'**elle se présente sous forme d'un gel, d'un liquide, d'une mousse.

18. Composition selon l'une quelconque des revendications précédentes, **caractérisée en ce qu'**elle est conditionnée dans un dispositif aérosol.

19. Procédé de traitement cosmétique des cheveux, **caractérisé en ce que** l'on applique la composition selon l'une quelconque des revendications précédentes sur les cheveux, et que l'on rince ou non après un éventuel temps de pose.

20. Utilisation de la composition selon l'une quelconque des revendications 1 à 18, comme produit de coiffage de préférence non rincé.
